# FASCICULE DE BREVET EUROPEEN

(11) **EP 1 254 247 B1**
(45) Date de publication et mention de la délivrance du brevet: **31.08.2005**
(21) Numéro de dépôt: 01907721.3
(22) Date de dépôt: 07.02.2001
(51) Int. Cl.: C12P 21/06, A61K 35/78, A61P 17/00

(54) **HYDROLYSAT DE PROTEINES ET DE BETA GLUCANES D'AVOINE UTILISABLE EN COSMETOLOGIE ET SON PROCEDE DE FABRICATION**
EIWEISS- UND BETA-GLUKANHYDROLYSAT AUS HAFER VERWENDBAR IN KOSMETOLOGIE UND HERSTELLUNGSVERFAHREN DAFÜR
PROTEIN AND OAT BETA GLUCAN HYDROLYSATE FOR USE IN COSMETOLOGY AND METHOD FOR MAKING SAME

(30) Priorité: 10.02.2000 FR 0001653
(43) Date de publication de la demande: 06.11.2002
(73) Titulaire: Pierre Fabre Dermo-Cosmetique, 92100 Boulogne-Billancourt (FR)
(72) Inventeur: DUMONT, Valérie, 31320 Castanet Tolosan (FR); ARIES, Marie-Françoise, F-31750 Escalquens (FR); TREBOSC, Marie-Thérèse, F-81100 Castres (FR); FABRE, Bernard, F-31450 Belberaud (FR)
(74) Mandataire: Ahner, Francis
(86) Numéro de dépôt international: PCT/FR2001/000355
(87) Numéro de publication internationale: WO 2001/059147

(56) Documents cités:
- EP-A- 0 619 950
- WO-A-89/08405

## Description

La présente invention concerne un hydrolysat de protéines et de β glucanes d'avoine, son procédé de fabrication, les compositions cosmétiques les comprenant et son utilisation comme apaisant pour les peaux hyper réactives et irritées.

L'avoine est une plante annuelle appartenant à la famille des graminées pouvant atteindre 1,50 mètre de haut. A la germination, la jeune plantule gazonne et donne plusieurs tiges qui sont des chaumes. Les feuilles sont alternes distiques de couleur glauque. Les épillets chez l'avoine sont pendants, grands et ne comprennent que deux fleurs. L'épillet porte à courte distance de son insertion, deux bractées particulières stériles, opposées, appelées glumes. Au-dessus des glumes l'axe de l'épillet porte 2 fleurs. A la base de chaque pédicelle floral on distingue deux formations foliacées qui sont des glumelles. Le fruit de l'avoine est un akène d'un type particulier appelé caryopse. C'est une graine allongée, effilée, pubescente, possédant un volumineux albumen amylacé. L'embryon possède un cotylédon très développé entourant presque complètement la tigelle. La graine est récoltée entourée de glumes et de glumelles. La graine se compose globalement de deux parties :
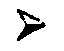 La partie externe de la graine, tégument, est riche en β glucanes
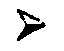 la partie interne de la graine contient l'amidon, les fractions lipidiques et glucoprotéines.

L'avoine est utilisée principalement sous la forme de farine à partir de la partie interne de la graine, et inscrite à la pharmacopée américaine sous l'appellation d'extrait colloïdal pour ses propriétés émollientes et adoucissantes. A partir de cette même partie, on peut extraire également de l'huile utilisée en cosmétologie et des protéines. Ces dernières, insolubles, ne sont pas utilisées directement mais après hydrolyse enzymatique ou chimique. On réalise un hydrolysat plus ou moins poussé qui permet d'obtenir, soit des peptides d'avoine de poids moléculaires variables, soit des acides aminés selon la force de l'hydrolyse. Les protéines d'avoine hydrolysées ont été examinées pour leurs propriétés dans le domaine cosmétique. Ainsi, il a été démontré des propriétés sur le cheveu comme la faculté qu'ont ces peptides à former un film sur la tige capillaire, de pénétrer dans la cuticule et ainsi par l'effet gainant résultant d'apporter un effet conditionneur (J. Hart, C. Polla, J. C. Hull, 1998, Cosmetics and Toiletries, 113, 40 45).

La partie externe de la graine fournit des β glucanes. Ces molécules sont des polysaccharides linéaires formés d'enchaînement de glucose avec des liaisons β 1,3 et β 1,4. Ces molécules sont utilisées en diététique dans les fibres d'avoine afin de réduire le taux de cholestérol et les risques cardiovasculaires. Cette application a même été retenue par la Food And Drug Administration (Fed Reg 62 (15) 3584 - 3601, amendement dans Fed Reg 62 (61) 15343 - 15344, 1977). Les β glucanes d'avoine ont été également étudiés pour leur propriété viscosante et gélifiante comme complément alimentaire (N. L. Dawkins and 1. A. Nnanna, 1995, Food Hydrocolloïds, 9,1, 1-7).

Les β glucanes ont montré des activités immunostimulantes (W. M. Kulicke, A. I. Lettau, H. Thielkins, 1997 Carbohydrate Research 297, 135-143), les β glucanes extraits de l'avoine présentent également cette activité (C. H. Yun, A. Estrada, A. V. Kessel, A. Gajadhar, M. Redmond and B. Laarveld, 1998, Microbiol Immunol 42 (6) 457-465). D'autres activités valorisées dans le domaine cosmétique ont été démontrées : protection contre les effets délétères des UV, stimulation du métabolisme cellulaire, stimulation de la synthèse du collagène et amélioration de la résistance aux tensions des cheveux (J. Hart, C. Polla, J. C. Hull, 1998, Cosmetics and Toiletries, 113, 40-45).

La préparation de fractions limpides d'hydrolysats d'avoine et leurs utilisations cosmétologiques et pharmacologiques ont été décrites dans EP 0 619 950.

Un hydrolysat enzymatique de protéines et de β glucanes d'avoine à partir des graines ou des sons d'avoine a été réalisé dans la présente invention et il a été démontré des activités stimulantes sur le TGF β1, cytokine immunosuppressive, régulatrice de l'inflammation et cicatrisante, chez le kératinocyte et inhibitrice sur la production des cytokines immunostimulantes Il2 et Il4 par le kératinocyte. Ces activités immunosuppressives et anti-inflammatoires dans un hydrolysat de protéines et de β glucanes sont originales.

La présente invention concerne un hydrolysat de protéines et de β glucanes susceptible d'être obtenu par :
a) mise en suspension dans de l'eau de graines d'avoine broyées ou de son d'avoine ou de farine d'avoine enrichie en son ;
b) acidification de la suspension et maintient du pH pendant au moins 20 minutes ;
c) neutralisation et légère alcalinisation de la suspension ;
d) hydrolyse de la suspension par ajout d'une protéase et d'une glucanase pendant au moins 4 heures ;
e) acidification de l'hydrolysat obtenu et maintient du pH pendant au moins 20 minutes ;
f) séparation des phases liquides des solides.

De préférence cet hydrolysat contient 10 à 20 % en peptides par rapport à la matière sèche et 55 à 75 % de sucres totaux par rapport à la matière sèche. De façon préférée, cet hydrolysat peut contenir 15 % de peptides par rapport à la matière sèche et 65 % de sucres totaux par rapport à la matière sèche ou 10 % de peptides par rapport à la matière sèche et 70 % de sucres totaux par rapport à la matière sèche. De façon encore plus préférée, cet hydrolysat contient 15 à 30 % en oligomères β glucanes par rapport à la matière sèche.

Cette invention concerne également un procédé de préparation de cet hydrolysat qui comporte les étapes de :
a) mise en suspension dans de l'eau de matière première constituée par des graines d'avoine broyées ou du son d'avoine ou de la farine d'avoine enrichie en son, le rapport matière première /eau pouvant varier de 1/50 à 1/5 ;
b) acidification de la suspension par addition d'acide, de préférence à un pH pouvant varier de 2 à 5, et maintient du pH pendant au moins 20 minutes, de préférence pendant 20 à 60 minutes, à une température pouvant varier de la température ambiante à 70°C ;
c) neutralisation et légère alcalinisation de la suspension par addition d'une base, de préférence à des valeurs de pH pouvant varier de 7 à 8,5 ;
d) hydrolyse de la suspension par ajout d'une protéase, de préférence en quantité de 0,02 parties de plantes, et d'une glucanase, de préférence en quantité de 0,002 parties de plantes, le temps d'hydrolyse pouvant varier de 4 à 10 heures et le pH et la température étant de préférence maintenue pendant toute la durée de l'hydrolyse;
e) acidification de l'hydrolysat obtenu par addition d'acide à un pH pouvant varier de 2 à 4, le pH et la température étant de préférence maintenue pendant 20 à 40 minutes ;
f) séparation des phases liquides des solides, de préférence par centrifugation, essorage ou filtration, pour obtenir un extrait aqueux limpide.

La mise en suspension de l'étape a) peut se faire sous agitation.

Le procédé peut comporter une étape supplémentaire de stabilisation au point de vue microbiologique de l'extrait aqueux limpide obtenu après l'étape f) par addition d'un conservateur microbien ou par addition de propylène ou butylène glycol.

De façon préférée, on évapore le solvant de l'extrait aqueux limpide obtenu après l'étape f) pour obtenir un extrait sec.

Des exemples d'acides utilisables dans le procédé sont l'acide sulfurique et l'acide acétique. Des exemples de bases sont la soude et la potasse.

Un exemple de protéase utilisable dans le procédé est la Neutrase® de chez Novo et des exemples de glucanases sont le Glucanex®, le Celluclast®, le Novozym® et l'Alcalase® de chez Novo.

Les quantités d'enzymes (0,02 et 0,002 parties de plantes) ajoutées sont calculées par rapport à la quantité de plantes (graines d'avoine broyées ou du son d'avoine ou de la farine d'avoine enrichie en son). Cette quantité est exprimée en partie (quantité) d'avoine engagée. Ainsi pour 1000 kg de plantes, on engagera par exemple 20 kg de protéase et 2 kg de glucanase.

Les hydrolysats d'avoine sont analysés pour leur teneur en peptides et en sucres totaux. Les peptides sont dosés selon la technique décrite à la Pharmacopée Européenne. Ce dosage est effectué après minéralisation de formes organiques de l'azote par le dosage de l'azote minérale. La teneur en peptides est obtenue par multiplication de la teneur en azote minéral par un coefficient moyen (6,25) qui représente la richesse en azote des protéines végétales. Les teneurs en peptides obtenues par rapport à la matière sèche varient de 10 à 20 %. Les sucres totaux sont dosés par la technique à l'anthrone, leur valeur par rapport à la matière sèche varie de 55 % à 75 %. Le dosage à l'anthrone est une méthode colorimétrique de dosage des sucres totaux après hydrolyse. Le réactif à l'anthrone est le 9,10-dihydro-9-oxoanthracène. Les sucres réagissent avec ce réactif pour donner une coloration à 585 nm. L'analyse des sucres totaux après hydrolyse enzymatique par une α glucanase et une β glucanase couplée à un dosage de sucres réducteurs a permis de déterminer la proportion des sucres liés en α ou en β. Ainsi nous pouvons préciser que 60 à 70 % des sucres sont des α glucanes et 30 à 40 % des β glucanes dans les oligomères. La teneur en oligomères β glucanes est donc de 15 à 30 % par rapport à la matière sèche.

La composition cosmétique, outre les oligomères de protéines et de β glucanes peut comprendre d'autres principes actifs conduisant à un effet complémentaire ou éventuellement synergique. On citera notamment les vitamines, les oligo-éléments, les dérivés protéiques, les huiles essentielles. Bien entendu cette liste n'est pas limitative et l'homme du métier pourra avec ses connaissances choisir d'autres matières actives qui en complément de la composition cosmétique selon l'invention produiront l'effet souhaité.

La composition selon l'invention est généralement une composition aqueuse constituée par de l'eau ou un mélange d'eau.

La composition cosmétique peut en outre renfermer au moins un adjuvant utilisé habituellement dans les compositions topiques destinées au soin de la peau. Parmi ces adjuvants, on peut citer les épaississants, les agents conservateurs, les agents antioxydants, les filtres solaires, les parfums, les colorants, les agents hydratants, les eaux thermales, les pigments minéraux etc.

La composition cosmétique peut se présenter sous la forme d'une crème, d'un masque, d'un lait, d'une lotion, d'un sérum, d'un spray, d'un dispositif transdermique ou d'un gel.

La présente invention concerne également l'utilisation des hydrolysats de protéines et de β glucanes et des compositions cosmétiques les comprenant comme stimulant de la production de TGF β1, comme inhibiteur des interleukines Il₂ et Il₄, comme agent immunosuppresseur des dermatoses inflammatoires et pour apaiser les peaux irritées ou hyper réactives.

L'invention est maintenant décrite en relation avec les exemples ci-après.

### Exemples de fabrication

### Exemple 1

1 kg de graines d'avoine broyées sont incorporées sous agitation dans 10 litres d'eau à 55°C. La suspension est amenée à pH 3 par rajout d'acide, l'agitation est maintenue pendant 30 minutes. Par rajout de base, le pH est amené à 8, on ajoute alors la protéase et la glucanase. L'agitation, la température et le pH initiaux sont maintenus pendant 6 heures. Après hydrolyse, le pH est abaissé à 3 par addition d'acide, la température et l'agitation sont maintenues pendant 30 minutes. L'hydrolysat est centrifugé, puis filtré. A cette solution on ajoute autant en poids de propylène glycol. La teneur en protéines est dosée à 15 % et celle des sucres totaux à 65 % par rapport à la matière sèche.

### Exemple 2

100 kg de son d'avoine sont placés dans 2 000 litres d'eau sous agitation à 40°C. Le pH est amené par addition d'acide à une valeur de 4,5. Le chauffage et l'agitation sont maintenus pendant 60 minutes. Le pH est ramené par addition de soude à 7,5, la protéase et la glucanase sont ajoutés. L'hydrolyse est effectuée pendant 10 heures à pH et température constants. Après hydrolyse le pH est placé à 3 par addition d'acide, la température et l'agitation sont maintenues pendant 30 minutes puis amenées à température ambiante. L'hydrolysat est alors centrifugé, filtré et concentré sous vide jusqu'à l'obtention d'une teneur en matière sèche de 60%. Le concentrat est fini d'être séché sous vide à 40°C en étuve. On obtient alors une poudre beige. La teneur en protéines de l'hydrolysat sec obtenu est de 10 %, les sucres totaux sont dosés à 70 %.

### Mise en évidence de l'activité de l'hydrolysat de protéines et de β glucanes d'avoine

Les oligomères de protéines et de β glucanes titrées à 15 % de peptides et 65% de sucres totaux ont été évalués sur la production de TGF β1, Il₂ et Il₄ par des kératinocytes.

### Evaluation sur le TGF β1

Le TGF β1 (Transforming Growth Factor - beta 1) appartient à la super famille de cytokines TGF P qui sont sécrétées par différents types cellulaires et qui jouent un rôle important dans le contrôle de la croissance cellulaire et la régulation de multiples réponses cellulaires et processus biologiques. Si de nombreuses cytokines capables de stimuler la prolifération cellulaire sont connues, le nombre de celles pourvues d'actions inhibitrices est plus restreint. C'est justement son effet inhibiteur de la croissance des cellules épithéliales et endothéliales qui permet de ranger le TGF β dans la catégorie des cytokines inhibitrices. Les cytokines de cette super famille possèdent trois activités majeures : elles inhibent la prolifération de la plupart des cellules (elles stimulent néanmoins la prolifération des fibroblastes), elles exercent des effets immunosuppresseurs, elles augmentent la formation de matrice extracellulaire (Transforming Growth Factor-beta : a general review Eur Cytokine Netw 1996, 7 (3) : 363-74).

Ainsi, les travaux de la littérature classent le TGF β 1 dans les cytokines anti-inflammatoires (Cytokines in inflammatory bowel disease World Surg 1998, 22 (4): 382-9). Au début d'une réponse inflammatoire, le TGF β 1, à des concentrations femtomolaires, attire les monocytes, les neutrophiles et les lymphocytes T par chimiotactisme et stimule leur production d'autres cytokines. Après l'activation de ces cellules aux sites de blessure ou de l'inflammation, l'action du TGF β 1, maintenant à des concentrations plus élevées, change vers une désactivation de ces cellules, un processus normal et nécessaire dans la réponse inflammatoire.

Le TGF β 1 joue également un rôle important dans l'immunorégulation notamment par ses effets immunosuppressifs qui sont largement décrits (Modulation of TGF β1 production from human keratinocytes by UVB Exp Dermatol 1 997, 6 (2): 1 50-10). Le TGF β 1 diminuerait l'activation des lymphocytes T en induisant une baisse d'expression des récepteurs pour l'interleukine 2, il jouerait un rôle régulateur négatif dans le développement des lymphocytes Th2, cellules préférentiellement activées chez les sujets atopiques (The TH1/TH2 paradigm in allergy Immunotechnology 1998, 3 (4): 233-44), et ceci notamment en limitant la sécrétion d'interleukine 4 (Regulation of human lymphocyte Il₄ secretion by intestinal epithelial cell-derived Il₇ ad TGF β Clin Immuno Immunopathol 1998, 88 (3): 287-96). Le TGF β serait capable de prédisposer les cellules présentatrices d'antigènes (CPA) à sécréter de l'interleukine 10 pendant le processing de l'antigène et de créer ainsi un "immune privilege" ou tolérance immune (A novel role for TGF-beta and IL₁₀ in the induction of immune privilege J Immunol 1998,160 (5): 2089-98), l'IL₁₀ étant capable d'inhiber les réponses Th1 et Th2 notamment de bloquer la production de cytokines par les Th1. Par ailleurs le TGFβ1 est impliqué dans la réparation des blessures, les processus de cicatrisation (Transforming Growth Factor-beta and wound healing. Int J. Biotechm Cell Biol 1997, 29 (1): 63-78) notamment en induisant une réorganisation du cytosquelette cellulaire (actine) et en promouvant la migration des cellules épithéliales (TGFβ1 promotes actin cytoskeleton reorganization and migratory phenotype in epithelial tracheal cells in primary culture. J. Cell Sci 1996, 109 (Pt9) : 2207-19).

Les oligomères de protéines et de β glucanes d'avoine ont été évalués sur la production par les kératinocytes du TGFβ1. Pour ce faire, les kératinocytes sont mis en culture puis exposés à différentes concentrations d'oligomères dans les milieux de culture. Après incubation, la production de TGFβ1 est mesurée par Kit Elisa.

3 concentrations d'oligomères ont été testées : 0,01 - 0,05 et 0,1 %.

Les oligomères de protéines et de β glucanes d'avoine présentent sur la production de TGF β1 des activités inductrices significatives, doses dépendantes :

| **Concentrations d'oligomères en %** | **Variation de production de TGFβ1 en %** |
|---|---|
| 0,05 | +18 |
| 0,1 | +33 |

Cette activité démontre l'intérêt des oligomères de protéines et de β glucanes d'avoine dans l'immunosuppression, l'inflammation et la cicatrisation.

### Evaluation sur les interleukines Il₂ et Il₄

Les mécanismes immunitaires qui jouent un rôle essentiel dans la pathogénèse de la dermatite atopique et d'autres dermatoses inflammatoires sont centrés autour de l'activation des lymphocytes T mais sont également la résultante d'interactions complexes entre plusieurs cellules dont les kératinocytes, les cellules endothéliales, les cellules de Langerhans, les éosinophiles, les lymphocytes T, et ceci par le biais de nombreux médiateurs dont les cytokines.

La dermatite atopique est caractérisée en partie par une dysrégulation de la production d'lgE et est associée à une infiltration locale de cellules lymphocytaires Th2. De nombreuses études montrent que les lymphocytes T de sujets atopiques présentent des profils de production de cytokines modifiés comparés à ceux de sujets sains et qu'ils sont des inducteurs potentiels de production d'lgE, ceci par leur production élevée d'interleukine 4 (Il₄) (Spontaneous expression of Il₄ mRNA in lymphocytes from children with atopic dermatitis - Clin Exp Immunol 1994, 97 (3): 491-8). Ces cellules induisent également la prolifération des éosinophiles (effecteurs importants des désordres allergiques) ceci par leur production élevée d'interleukine 5 (Il₅).

La réponse cellulaire des lymphocytes T dans la pathogenèse de la dermatite atopique continue à être bien explorée et il est maintenant établi que les lymphocytes T helper 1 et 2 (Th1 et Th2) jouent ensemble un rôle dans le processus de la maladie mais à des stades différents. Les Th2 semblent être importants dans les stades précoces du développement de lésion alors que les Th1 seraient impliqués plus tardivement (Atopic dermatitis - J Eur Acad Dermatol Venereol 1996 (7) 101-114).

Cet environnement de désordres immunitaires justifie le choix d'agents potentiellement immunosuppresseurs en terme de thérapeutique, agents inhibant la transcription des gènes codant pour l'interleukine 2, l'interféron y, l'interleukine 4, l'interleukine 5, donc les gènes des cytokines des deux sous-populations de lymphocytes T, les Th1 et les Th2 (Tacrolimus (FK506): Experience in dermatology Dermatologic Therapy, 1998, vol 5 : 74-84) (Regulation of interleukin 5 production by peripheral blood mononuclear cells from atopic patients with FK506, cyclosporin A and glucocorticoids - Int Arch Allergy Immunol 1994, 104 Suppl 1 (1): 32-5. Le raisonnement scientifique est maintenant bien clair en ce qui concerne l'utilisation d'agents immunomodulateurs ou immunosuppresseurs pour traiter les dermatoses inflammatoires et les études récentes réalisées dans cette optique sont très prometteuses et supportent le concept postulant que les thérapies qui modulent les fonctions des cellules T et leur production de cytokines sont efficaces pour réduire la sévérité clinique de la dermatite atopique (Atopic dermatitis : immunobiology and treatment with immune modulators - Clin Exp Immunol, 1997, 107 (Suppl. 1) : 25-30.

Les oligomères de protéines et de β glucanes ont été évalués sur la production in vitro d'interleukine 2 et interleukine 4 par les cellules mononuclées humaines. Pour ce faire, les cellules mononuclées (lymphocytes et monocytes) sont mises en suspension dans un milieu de culture approprié puis exposées à différentes concentrations d'oligomères (0,01 % - 0,05 % - 0,1 %) puis stimulées pour la production d'Il₂ et Il₄. Ces dernières, après incubation des cellules et récupération de surnageants cellulaires sont dosées par Kit Elisa.

Les oligomères d'avoine inhibent significativement et de façon dose dépendante l'interleukine Il₂ et l'interleukine Il₄:

| **Concentrations d'oligomères en %** | **Variation de la production d'Il**_{**2**} **en %** | **Variation de la production d'Il**_{**4**} **en %** |
|---|---|---|
| 0,01 | -20 | -9 |
| 0,05 | -47 | -37 |
| 0,1 | -93 | -91 |

Par cette activité nous avons démontré l'activité immunosuppressive surprenante des oligomères de protéines et β glucanes d'avoine.

### Crème pour peau sensible et hyper réactive

- Oligomères d'avoine 0,01 à 5 %
- Vitamine E acétate 0,5 %
- Prépolyol polymère 0,5 %
- Sucrose distéarate 5 %
- Triglycéride caprique/caprylique 11 %
- Vaseline blanche 3 %
- Pérhydrosqualane 5 %
- Polymère carboxyvinylique 0,2 %
- Tréthanolamine 0,01 %
- Butylène glycol 2 %
- Conservateurs q.s.
- Eau potable q.s.p. 100 g

Cette formule peut aussi contenir des pigments minéraux et faire office de crème teintée.

### Masque apaisant

- Oligomères d'avoine 0,5 à 5 %
- Extrait colloïdal d'avoine 3 %
- Lécithine de soja 2 %
   (Prolipo H de la société Lucas Meyer)
- Gomme xanthane 0,3 %
- Sepigel 305 0,4 %
- Conservateurs q.s.
- Eau potable q.s.p. 100 g

### Lotion apaisante pour peaux hyper réactives

- Oligomères d'avoine 0,1 à 10 %
- Tanins de châtaignier 0,1 à 0,5 %
- Eux florales 5 à 10 %
- Gomme xanthane 0,1 %
- Glycérine 2 %
- Conservateurs q.s.
- Eau potable q.s.p.100 g

### Lait corporel peaux sèches, irritées

- Oligomères d'avoine 0,1 à 10 %
- Huile de bourrache 2 %
   ou Huile d'onagre 10 %
- Vaseline blanche 5 %
- Octyl palmitate 7 %
- Monostéarate de glycérol 5 %
   Auto Emulsionnable
- Pémulen/TR1 0,4 %
- Triéthanolamine 0,4 %
- Alcool benzylique 1 %
- Conservateurs q.s.
- Eau potable q.s.p. 100 g

Quelques modèles type de formule ont été décrits. Il est évident que la liste n'est pas limitative et qu'elle concerne toute forme galénique à usage topique pour le soin de la peau.

## Revendications

1. Extrait aqueux limpide d'hydrolysat de protéines et de β glucanes susceptible d'être obtenu par :
a) mise en suspension dans de l'eau de graines d'avoine broyées ou de son d'avoine ou de farine d'avoine enrichie en son;
b) acidification de la suspension et maintient du pH pendant au moins 20 minutes;
c) neutralisation et légère alcalinisation de la suspension;
d) hydrolyse de la suspension par ajout d'une protéase et d'une glucanase pendant au moins 4 heures;
e) acidification de l'hydrolysat obtenu et maintient du pH pendant au moins 20 minutes;
f) séparation des phases liquides des solides
et contenant 10 à 20 % en peptides par rapport à la matière sèche et 55 à 75 % en sucres totaux par rapport à la matière sèche.

2. Extrait selon la revendication 1, **caractérisé en ce qu'**il contient 15 % en peptides par rapport à la matière sèche et 65 % de sucres totaux par rapport à la matière sèche.

3. Extrait selon les revendications 1, **caractérisé en ce qu'**il contient 10 % de peptides par rapport à la matière sèche et 70 % de sucres totaux par rapport à la matière sèche.

4. Extrait selon l'une quelconque des revendications précédentes, **caractérisé en ce qu'**il contient 15 à 30 % en oligomères β glucanes par rapport à la matière sèche.

5. Procédé de préparation d'un extrait selon les revendications 1 à 4, **caractérisé en ce qu'**il comporte les étapes de :
a) mise en suspension dans de l'eau de graines d'avoine broyées ou de son d'avoine ou de farine d'avoine enrichie en son ;
b) acidification de la suspension et maintient du pH pendant au moins 20 minutes ;
c) neutralisation et légère alcalinisation de la suspension ;
d) hydrolyse de la suspension par ajout d'une protéase et d'une glucanase pendant au moins 4 heures ;
e) acidification de l'hydrolysat obtenu et maintient du pH pendant au moins 20 minutes ;
f) séparation des phases liquides et solides.

6. Procédé de préparation selon la revendication 5, **caractérisé en ce que** la mise en suspension de l'étape a) se fait sous agitation.

7. Procédé selon les revendications 5 et 6 **caractérisé en ce que** le rapport avoine/eau varie de 1/50 à 1/5.

8. Procédé selon l'une quelconque des revendications 5 à 7, **caractérisé en ce que** le pH de l'étape b) peut varier de 2 à 5.

9. Procédé selon l'une quelconque des revendications 5 à 8, **caractérisé en ce que** le pH acide de l'étape b) est maintenu pendant 20 à 60 minutes.

10. Procédé selon l'une quelconque des revendications 6 à 9, **caractérisé en ce que** la température de l'étape b) peut varier de la température ambiante à 70°C.

11. Procédé selon l'une quelconque des revendications 5 à 10, **caractérisé en ce que** le pH de l'étape c) peut varier de 7 à 8,5.

12. Procédé selon l'une quelconque des revendications 5 à 11, **caractérisé en ce que** dans l'étape d) la quantité de protéase utilisée est de 0,02 parties de plantes et celle de glucanase est de 0,002 parties de plantes.

13. Procédé selon l'une quelconque des revendications 5 à 12, **caractérisé en ce que** le temps d'hydrolyse de l'étape d) peut varier de 4 à 10 heures.

14. Procédé selon l'une quelconque des revendications 5 à 13, **caractérisé en ce que** dans l'étape d) le pH et la température sont maintenus pendant toute la durée de l'hydrolyse.

15. Procédé selon l'une quelconque des revendications 5 à 14, **caractérisé en ce que** le pH de l'étape e) peut varier de 2 à 4.

16. Procédé selon l'une quelconque des revendications 5 à 15, **caractérisé en ce que** la température de l'étape e) est maintenue de 20 à 40 minutes.

17. Procédé selon l'une quelconque des revendications 5 à 16, **caractérisé en ce que** l'étape f) se fait par centrifugation, essorage ou filtration.

18. Procédé selon l'une quelconque des revendications 5 à 17, **caractérisé en ce que** l'on stabilise au point de vue microbiologique l'extrait aqueux limpide obtenu après l'étape f) par addition d'un conservateur microbien ou par addition de propylène ou butylène glycol.

19. Procédé selon l'une quelconque des revendications 5 à 18, **caractérisé en ce qu'**on évapore le solvant de l'extrait aqueux limpide obtenu après l'étape f) pour obtenir un extrait sec.

20. Composition pharmaceutique ou cosmétique comprenant un des composés selon l'une quelconque des revendications 1 à 4 et un excipient approprié.

21. Composition pharmaceutique ou cosmétique selon la revendication 20, **caractérisée en ce qu'**elle se présente sous la forme d'une crème, d'un masque, d'une lotion, d'un lait, d'un gel, d'un spray, d'un sérum ou d'un dispositif transdermique.

22. A titre de médicament, les produits selon les revendications 1 à 4, 20 et 21.

23. A titre de médicament stimulant la production de TGF β1, les produits selon les revendications 1 à 4, 20 et 21.

24. A titre de médicament inhibant les interleukines Il₂ et Il₄, les produits selon les revendications 1 à 4, 20 et 21.

25. A titre de médicament immunosuppresseur des dermatoses inflammatoires, les produits selon les revendications 1 à 4, 20 et 21.

26. A titre de médicament apaisant pour les peaux irritées ou hyper réactives, les produits selon les revendications 1 à 4, 20 et 21.

## Patentansprüche

1. Klarer wässriger Extrakt eines Hydrolysats von Proteinen und von β-Glucanen, welcher erhalten werden kann durch:
a) Suspendieren von zermahlenen Haferkörnern oder von Haferkleie oder von mit Kleie angereichertem Hafermehl in Wasser;
b) Ansäuern der Suspension und Beibehalten des pH während wenigstens 20 min;
c) Neutralisation und leichtes Alkalischmachen der Suspension;
d) Hydrolyse der Suspension durch Zugabe einer Protease und einer Glucanase während wenigstens 4 h;
e) Ansäuern des erhaltenen Hydrolysats und Beibehalten des pH während wenigstens 20 min;
f) Abtrennung der flüssigen Phasen von den Feststoffen
und welcher 10 bis 20% Peptide bezogen auf die Trockensubstanz und 55 bis 75% an gesamten Zuckern bezogen auf die Trockensubstanz enthält.

2. Extrakt nach Anspruch 1, **dadurch gekennzeichnet, dass** er 15% Peptide bezogen auf die Trockensubstanz und 65% an gesamten Zuckern bezogen auf die Trockensubstanz enthält.

3. Extrakt nach Anspruch 1, **dadurch gekennzeichnet, dass** er 10% Peptide bezogen auf die Trockensubstanz und 70% an gesamten Zuckern bezogen auf die Trockensubstanz enthält.

4. Extrakt nach einem der vorangegangenen Ansprüche, **dadurch gekennzeichnet, dass** er.15 bis 30% an β-Glucan-Oligomeren bezogen auf die Trockensubstanz enthält.

5. Verfahren zur Herstellung eines Extrakts nach den Ansprüchen 1 bis 4, **dadurch gekennzeichnet, dass** es die Schritte umfasst:
a) Suspendieren von zermahlenen Haferkörnern oder von Haferkleie oder von mit Kleie angereichertem Hafermehl in Wasser;
b) Ansäuern der Suspension und Beibehalten des pH während wenigstens 20 min;
c) Neutralisation und leichtes Alkalischmachen der Suspension;
d) Hydrolyse der Suspension durch Zugabe einer Protease und einer Glucanase während wenigstens 4 h;
e) Ansäuern des erhaltenen Hydrolysats und Beibehalten des pH während wenigstens 20 min;
f) Trennung der flüssigen und festen Phasen.

6. Herstellungsverfahren nach Anspruch 5, **dadurch gekennzeichnet, dass** das Suspendieren des Schritts a) unter Bewegung erfolgt.

7. Verfahren nach den Ansprüchen 5 und 6, **dadurch gekennzeichnet, dass** das Verhältnis Hafer/Wasser von 1/50 bis 1/5 variiert.

8. Verfahren nach einem der Ansprüche 5 bis 7, **dadurch gekennzeichnet, dass** der pH des Schritts b) von 2 bis 5 variieren kann.

9. Verfahren nach einem der Ansprüche 5 bis 8, **dadurch gekennzeichnet, dass** der saure pH des Schritts b) während 20 bis 60 min beibehalten wird.

10. Verfahren nach einem der Ansprüche 6 bis 9, **dadurch gekennzeichnet, dass** die Temperatur des Schritts b) von Umgebungstemperatur bis 70°C variieren kann.

11. Verfahren nach einem der Ansprüche 5 bis 10, **dadurch gekennzeichnet, dass** der pH des Schritts c) von 7 bis 8,5 variieren kann.

12. Verfahren nach einem der Ansprüche 5 bis 11, **dadurch gekennzeichnet, dass** in dem Schritt d) die eingesetzte Proteasemenge 0,02 Pflanzenteile und jene von Glucanase 0,002 Pflanzenteile beträgt.

13. Verfahren nach einem der Ansprüche 5 bis 12, **dadurch gekennzeichnet, dass** die Hydrolysedauer des Schritts d) von 4 bis 10 h variieren kann.

14. Verfahren nach einem der Ansprüche 5 bis 13, **dadurch gekennzeichnet, dass** in dem Schritt d) der pH und die Temperatur während der gesamten Dauer der Hydrolyse beibehalten werden.

15. Verfahren nach einem der Ansprüche 5 bis 14, **dadurch gekennzeichnet, dass** der pH des Schritts e) von 2 bis 4 variieren kann.

16. Verfahren nach einem der Ansprüche 5 bis 15, **dadurch gekennzeichnet, dass** die Temperatur des Schritts e) 20 bis 40 min beibehalten wird.

17. Verfahren nach einem der Ansprüche 5 bis 16, **dadurch gekennzeichnet, dass** der Schritt f) durch Zentrifugation, Schleudern/Wringen oder Filtration erfolgt.

18. Verfahren nach einem der Ansprüche 5 bis 17, **dadurch gekennzeichnet, dass** man den klaren wässrigen Extrakt, der nach dem Schritt f) erhalten wird, durch Zugabe eines mikrobiellen Konservierungsmittels oder durch Zugabe von Propylen- oder Butylenglycol aus mikrobiologischer Sicht stabilisiert.

19. Verfahren nach einem der Ansprüche 5 bis 18, **dadurch gekennzeichnet, dass** man das Lösemittel des nach dem Schritt f) erhaltenen klaren wässrigen Extrakts verdampft, um einen Trockenauszug zu erhalten.

20. Pharmazeutische oder kosmetische Zusammensetzung, welche eine der Verbindungen nach einem der Ansprüche 1 bis 4 und ein geeignetes Vehikel umfasst.

21. Pharmazeutische oder kosmetische Zusammensetzung nach Anspruch 20, **dadurch gekennzeichnet, dass** sie in Form einer Creme, einer Maske, einer Lotion, einer Milch, eines Gels, eines Sprays, eines Serums oder einer transdermalen Vorrichtung vorliegt.

22. Erzeugnisse nach den Ansprüchen 1 bis 4, 20 und 21 als Arzneimittel.

23. Erzeugnisse nach den Ansprüchen 1 bis 4, 20 und 21 als Arzneimittel, welches die Produktion von TGF-β1 stimuliert.

24. Erzeugnisse nach den Ansprüchen 1 bis 4, 20 und 21 als Arzneimittel, welches die Interleukine Il₂ und Il₄ inhibiert.

25. Erzeugnisse nach den Ansprüchen 1 bis 4, 20 und 21 als immunsuppressives Arzneimittel für entzündliche Dermatosen.

26. Erzeugnisse nach den Ansprüchen 1 bis 4, 20 und 21 als beruhigend wirkendes Arzneimittel für die gereizte oder hyperreaktive Haut.

## Claims

1. Clear aqueous extract of protein and β-glucan hydrolysate obtainable by:
a) suspending ground oat grains or oat bran or bran-enriched oat flour in water;
b) acidifying the suspension and maintaining the pH for at least 20 minutes;
c) neutralizing and slightly alkalizing the suspension;
d) hydrolysing the suspension by adding a protease and a glucanase for at least 4 hours;
e) acidifying the hydrolysate obtained and maintaining the pH for at least 20 minutes;
f) separating the liquid and solid phases
and containing 10 to 20% of peptides relative to the dry matter content and 55 to 75% of total sugars relative to the dry matter content.

2. Extract according to Claim 1, **characterized in that** it contains 15% of peptides relative to the dry matter content and 65% of total sugars relative to the dry matter content.

3. Extract according to Claim 1, **characterized in that** it contains 10% of peptides relative to the dry matter content and 70% of total sugars relative to the dry matter content.

4. Extract according to any one of the preceding claims, **characterized in that** it contains 15 to 30% of β-glucan oligomers relative to the dry matter content.

5. Method for preparing an extract according to Claims 1 to 4, **characterized in that** it comprises the steps consisting in:
a) suspending ground oat grains or oat bran or bran-enriched oat flour in water;
b) acidifying the suspension and maintaining the pH for at least 20 minutes;
c) neutralizing and slightly alkalizing the suspension;
d) hydrolysing the suspension by adding a protease and a glucanase for at least 4 hours;
e) acidifying the hydrolysate obtained and maintaining the pH for at least 20 minutes;
f) separating the liquid and solid phases.

6. Method of preparation according to Claim 5, **characterized in that** the suspension in step a) is performed with stirring.

7. Method according to Claims 5 and 6, **characterized in that** the oat/water ratio varies from 1/50 to 1/5.

8. Method according to any one of Claims 5 to 7, **characterized in that** the pH in step b) may vary from 2 to 5.

9. Method according to any one of Claims 5 to 8, **characterized in that** the acidic pH in step b) is maintained for 20 to 60 minutes.

10. Method according to any one of Claims 6 to 9, **characterized in that** the temperature in step b) may vary from room temperature to 70°C.

11. Method according to any one of Claims 5 to 10, **characterized in that** the pH in step c) may vary from 7 to 8.5.

12. Method according to any one of Claims 5 to 11, **characterized in that** in step d), the quantity of protease used is 0.02 plant parts and that of glucanase is 0.002 plant parts.

13. Method according to any one of Claims 5 to 12, **characterized in that** the hydrolysis time in step d) may vary from 4 to 10 hours.

14. Method according to any one of Claims 5 to 13, **characterized in that** in step d), the pH and the temperature are maintained during the entire duration of the hydrolysis.

15. Method according to any one of Claims 5 to 14, **characterized in that** the pH in step e) may vary from 2 to 4.

16. Method according to any one of Claims 5 to 15, **characterized in that** the temperature in step e) is maintained for 20 to 40 minutes.

17. Method according to any one of Claims 5 to 16, **characterized in that** step f) is performed by centrifugation, draining or filtration.

18. Method according to any one of Claims 5 to 17, **characterized in that** the clear aqueous extract obtained after step f) is microbiologically stabilized by adding a microbial preservative or by adding propylene or butylene glycol.

19. Method according to any one of Claims 5 to 18, **characterized in that** the solvent is evaporated from the clear aqueous extract obtained after step f) in order to obtain a dry extract.

20. Pharmaceutical or cosmetic composition comprising one of the compounds according to any one of Claims 1 to 4 and an appropriate excipient.

21. Pharmaceutical or cosmetic composition according to Claim 20, **characterized in that** it is in the form of a cream, a face pack, a lotion, a milk, a gel, a spray, a serum or a patch.

22. As a medicament, the products according to Claims 1 to 4, 20 and 21.

23. As a medicament stimulating the production of TGF-β1, the products according to Claims 1 to 4, 20 and 21.

24. As a medicament inhibiting interleukins II₂ and II₄, the products according to Claims 1 to 4, 20 and 21.

25. As a medicament for the immunosuppression of inflammatory dermatoses, the products according to Claims 1 to 4, 20 and 21.

26. As a soothing medicament for irritated or hyper-reactive skin, the products according to Claims 1 to 4, 20 and 21.
